# EUROPEAN PATENT SPECIFICATION

(11) **EP 1 131 009 B1**
(45) Date of publication and mention of the grant of the patent: **09.02.2005**
(21) Application number: 98958603.7
(22) Date of filing: 16.11.1998
(51) Int. Cl.: A61B 18/14

(54) **MULTIFUNCTIONAL TELESCOPIC ELECTROSURGICAL INSTRUMENT**
MULTIFUNKTIONALE TELESKOPISCHE ELEKTROCHIRURGISCHE VORRICHTUNG
INSTRUMENT ELECTROCHIRURGICAL MULTIFONCTIONS TELESCOPIQUE

(43) Date of publication of application: 12.09.2001
(73) Proprietor: Cosmescu, Ioan, Phoenix, Arizona 85027 (US)
(72) Inventor: Cosmescu, Ioan, Phoenix, Arizona 85027 (US)
(74) Representative: Hanna, Peter William Derek
(86) International application number: PCT/US1998/024434
(87) International publication number: WO 2000/028908

(56) References cited:
- EP-A- 0 649 634
- US-A- 5 234 429
- US-A- 5 254 117
- US-A- 5 281 216
- US-A- 5 423 804
- US-A- 5 496 314
- US-A- 5 693 044

## Description

### BACKGROUND OF THE INVENTION

### Field of the invention

The present invention relates generally to an electrosurgical unit (ESU) pencil. More specifically, the present invention relates to an ESU pencil having a bipolar electrode wherein the active and return electrode are contained within the same tip of the ESU pencil and are separated by a high temperature resistant radio frequency (RF) dielectric. The ESU pencil having a bipolar electrode is designed for use with a monopolar ESU device in a bipolar function for cutting and coagulation in medical procedures. When used with a monopolar ESU device, the ESU pencil having a bipolar electrode can also be used for monopolar application wherein a separate electrode is applied to a part of the patient's body, usually on the patients leg, to function as the return electrode. This monopolar function is prevalent in the prior art.

The present invention also relates to a telescopic laparoscopic monopolar/bipolar ESU pencil having an adjustable length electrode capable of accommodating different depths and/or different sizes of adult and children. The telescopic laparoscopic monopolar/bipolar ESU pencil can also be used with an integrated smoke evacuator system such as that previously described in U.S. Patent Number 51199,944. Also, the ESU pencil of the present invention having a bipolar electrode may be combined with the suction/irrigation system and apparatus previously described in my co-pending patent application entitled "Automatic Suction/Irrigation Apparatus for Use in Laparoscopic Surgery and Electrosurgery and Method Therefor" which is being simultaneously filed with the present application and is incorporated by reference thereto. Finally, the ESU pencil of the present invention having a bipolar electrode can be combined with attachments so that it can also be used for argon beam coagulation,

### Description of the Prior Art

In the past, electrosurgical devices had an active electrode that was used for performing cutting and coagulation, and a return electrode which usually comprised an adhesive for attachment to a patient's skin. When the electrosurgery unit surgery pencil was activated, the RF energy circulated from the active electrode to the return electrode through the patient's body with the distances between the active and return electrodes being fairly significant.

This prior art system presents several deficiencies and creates a number of problems that can be dangerous for the patient. First, because of the significant distance between the active and return electrodes, high voltages at high frequencies traverse through the body in order to close the circuit between the active and return electrodes. These high voltages and frequencies can be very dangerous to the patient. Second, due to the fact that the body has an electrical resistance, the cutting and coagulation affects are significantly diminished as the distance between the active and return electrodes is increased. Accordingly, high power from the ESU is needed in order to obtain effective cutting and coagulation performance. Again, the high power required from the ESU can be dangerous to the patient

Third, in many cases, patients have incurred significant burns because of poor contact between the return electrode and the patiant's skin. Finally, dangerous capacitive coupling has occurred in laparoscopic surgery which has resulted in dangerous burns to the patient. These bums could not be detected because they are out of the field of view of the laparoscope thereby putting the patient's life in danger.

A bipolar function for open and laparoscopic procedures is presently being used with the bipolar suction of the ESU. However, this system and the related instruments are used only to stop bleeding within a patient and is not capable of performing a cutting operation.

Other prior art references include U.S. Patent No. 5,495,314 which is directed to an electrically powered endoscopic probe capable of supplying suction and irrigation to a surgical site. The probe may employ a bipolar electrosurgical probe tip and may include an extendable shroud for selectively covering the tip of the prove while positioning or manipulating the prove at the surgical site. Further, U.S. Patent No. 5,218,216 discloses an electrosurgical apparatus having a bipolar electrode attached to a handpiece.

The apparatus of the present invention as defined in claim 1 provides a surgeon with easier access to the coagulation with an electrosurgery instrument. Applicant's apparatus specifically provides for a telescopic electrical surgical pencil having an electrical connection to the electrode located along a length of the telescopic apparatus thereby enabling a surgeon to extend the electrode a great distance to position it at a surgical site while simultaneously extending a tubular member positioned around the electrode which functions to provide suction and/or irrigation.

As previously stated in the section referred to as "related application", the present invention is related to the same invantor's pending patent application entitled "Telescopic Surgical Device and Method Therefor. This similarities between the telescopic pencil described in the pending application and the present invention described in this application is that both inventions refer to a telescopic pencil which can have multifunctional applications. The main difference between the inventions is that the pending telescopic pencil patent application refers to a monopolar pencil only which has only one contact for the electrode while the ESU pencil of the present invention described in this application can be used as a monopolar instrument, a monopolar/bipolar instrument or a bipolar instrument The ESU pencil of the present invention has an electrode which comprises two contacts, one which is used as an active electrode and another which is used as a return electrode when the instrument is used as a bipolar functioning instrument for a monopolar/bipolar functioning instrument. Alternatively, when the instrument is only used as a monopolar functioning instrument, a separate electrode is applied to a different part of the patient's body, usually on the patients leg. This separate electrode functions as the return electrode.

### SUMMARY OF THE INVENTION

A principal object of the present invention is to provide an electrosurgery electrode for performing cutting and coagulation for open and closed endoscopic and laparoscopic procedures wherein the electrosurgery electrode contains both the active electrode and the return electrode on the same tip.

It is a further objection of the present invention to provide a monopolar/bipolar electrode which can be used on the monopolar section of an electrosurgery unit to perform a bipolar function thereby eliminating the need for a separate return electrode.

It is still a further object of the present invention to provide a telescopic monopolar and monopolar/bipolar electrode and pencil with smoke evacuation means wherein the distance between the operating tip of the electrode and the hand piece is adjustable to accommodate desired lengths associated with different sized patients.

It is yet a further object of the present invention to provide a telescopic monopolar and monopolar/bipolar endoscopic and laparoscopic electrode, with or without smoke evacuation means, wherein the length of the laparoscopic electrode is adjustable.

It is still a further object of the present invention to provide a telescopic monopolar and monopolar/bipolar ESU pencil with suction/irrigation means wherein the bipolar electrode is automatically retracted upon activation of the suctioning means.

It is still a further object of the present invention to provide a telescopic monopolar and monopolar/bipolar electrode and pencil for open and closed endoscopic and laparoscopic procedures with suction/irrigation means wherein the electrode can be automatically retracted upon the activation of the suctioning means.

It is yet a further object of the present invention to provide a multifunctional telescopic ESU pencil that can be used for open and laparoscopic electrosurgery that is also capable of performing suction and irrigation, and for argon beam coagulation when used with respective attachments as previously described in reference to my U.S. Patent US-A-5,693,044 entitled "Telescopic Surgical Device and Method Therefor".

Accordingly, the multifunctional telescopic monopolar/bipolar surgical device of the present invention is defined in claim 1, and includes a bipolar electrode and a hand piece having electrical contacts wherein the bipolar electrode is connected to the electrical contacts of the hand piece and the electrical contacts of the hand piece are connected to an energy source for activating the device. An electrosurgery unit may be used as the energy source. The bipolar electrode includes an active electrode, a return electrode and an insulator which is sandwiched between the active and return electrodes. The multifunctional telescopic monopolar/bipolar surgical device further includes a telescopic member coupled to the bipolar electrode and the hand piece for adjusting the length of the bipolar electrode. Further, the multifunctional telescopic monopolar/bipolar surgical device may include a smoke evacuation means coupled to the bipolar electrode for removing smoke and other debris that is produced during electrosurgery.

The present invention is also directed to a telescopic suction/irrigation apparatus for open and endoscopic laparoscopic procedures which includes a hollow hand piece member having an open end and an open proximal end and connection means for connecting the hand piece to an energy source for activating suction and irrigation, an elongated hollow tubular member having distal and proximal open ends wherein the proximal open end is introduced into the open distal end of the hand piece so that the elongated hollow tubular members concentrically contained within the channel of the hand piece and locking means for locking the elongated hollow tubular member within the hand piece. Another embodiment of the telescopic suction/irrigation apparatus comprises a hand piece member having connection means to an energy source and means for effectuating suction and irrigation functions, a double channel telescopic suction/irrigation tube having an inner channel and outer channel wherein the double channel telescopic suction/irrigation tube is introduced into the hand piece member such that a portion of the double channel telescopic suction/irrigation tube is concentrically retained within the hand piece, connection means for connecting the inner channel of said double channel telescopic suction/irrigation tube with an irrigation port and the outer channel of the double channeled telescopic suction/irrigation tube with a suctioning port, and locking means for locking the double channel telescopic suction/irrigation tube within the hand piece member.

The foregoing and other objects, features and advantages of the present invention, as well as details of the preferred embodiments thereof, will be more fully understood from the following description made in conjunction with the accompanying drawings.

### BRIEF DESCRIPTION OF THE DRAWINGS

FIG. 1a is a diagrammatic view of electrosurgery being performed on a patient using a prior art ESU pencil having a monopolar electrode which requires a separate return electrode.
FIG. 1b is a diagrammatic view of electrosurgery being performed on a patient using the telescopic monopolar/bipolar surgical device of the present invention for electrosurgery.
FIG. 2a is a perspective view of a first embodiment of a bipolar electrode for use within the telescopic monopolar/bipolar surgical device of the present invention for electrosurgery which can also function as a monopolar electrode.
FIG. 2b is a perspective view of a second embodiment of a bipolar electrode for use within the telescopic monopolar/bipolar surgical device of the present invention for electrosurgery which can also function as a monopolar electrode.
FIG. 2c is a perspective view of a third embodiment of a bipolar electrode for use within the telescopic monopolar/bipolar surgical device of the present invention for electrosurgery which can also function as a monopolar electrode.
FIG. 2d is a perspective view of a first embodiment of a bipolar electrode used in the multifunctional telescopic monopolar/bipolar surgical device of the present invention for endoscopic and/or laparoscopic procedures wherein the bipolar electrode is capable of a monopolar function.
FIG. 2e is a perspective view of a second embodiment of a bipolar electrode used in the multifunctional telescopic monopolar/bipolar surgical device of the present invention for endoscopic and/or laparoscopic procedures wherein the bipolar electrode is capable of a monopolar function.
FIG. 2f is a perspective view of a third embodiment of a bipolar electrode used in the multifunctional teiescopic monopolar/bipolar surgical device of the present invention for endoscopic and/or laparoscopic procedures wherein the bipolar electrode is capable of a monopolar function.
FIG. 3a is a perspective view of the multifunctional monopolar/bipolar telescopic electrosurgical device of the present invention.
FIG. 3b is a perspective exploded view of the device in FIG. 3a shown without the electrical cord.
FIG. 3c is cross-sectional view of the device in FIG. 3a shown without the electrical cord.
FIG. 3d is perspective view of an endoscopic and/or laparoscopic telescope element which can replace the telescopic element of the multifunctional monopolar/bipolar telescopic electrosurgical device shown in FIG. 3c to create a multifunctional monopolar/bipolar telescopic electrosurgical device for endoscopic and/or laparoscopic procedures.
FIG. 4a is a perspective view of a suction/irrigation telescope which can replace the electrosurgery telescope in FIG. 3c or the laparoscopic telescope in FiG. 3d to provide a telescopic surgical device with suction/irrigation means capable of performing either suction or irrigation.
FIG. 4b is a perspective exploded view and partial cross-sectional view of a second embodiment of a suction/irrigation telescope which can replace the electrosurgery telescope in FIG. 3c or the laparoscopic telescope in FIG. 3d to provide a telescopic device with suction/irrigation means capable of performing suction and irrigation simultaneously.
FIG. 4c is a partial cross-sectional view of a hydro dissection nozzle wherein the internal tube of the nozzle is shown retracted.
FIG. 4d is a partial cross-sectional view of the hydro dissection nozzle shown in FIG. 4c with the internal tube shown extended.

### DESCRIPTION OF THE PREFERRED EMBODIMENTS

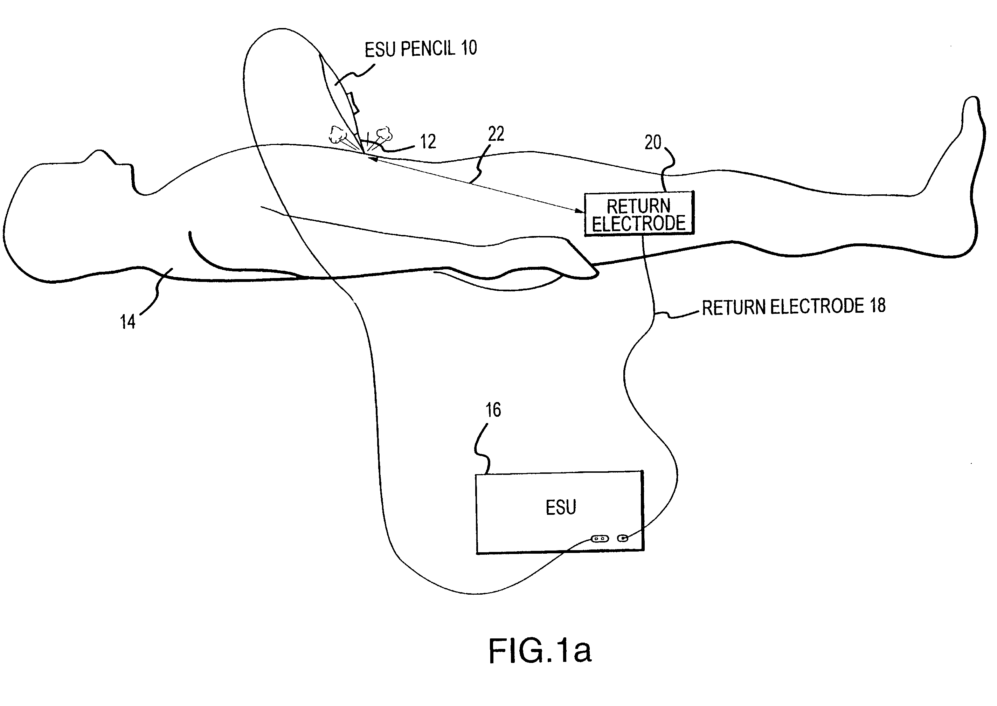

The present system for performing electrosurgery with an ESU pencil having a monopolar electrode is shown in FIG. 1a. The electrosurgery pencil 10 comprises an active electrode 12 which performs cutting or coagulation on a patient 14. The ESU pencil 10 comprising active electrode 12 is connected to the electrosurgery unit 16 which provides the energy source to activate the ESU pencil 10. A return electrode 18 comprises an adhesive patch 20 which is positioned and applied to the patient 14 at a considerable distance 22 away from the active electrode 12. The return electrode 1 8 is also connected to the electrosurgery unit 16 thereby creating a closed circuit wherein the voltage and frequency emitted from the active electrode 12 is transmitted through the body of the patient 14 and received by return electrode 20. FIG. 1a illustrates how monopolar electrosurgery is presently performed.

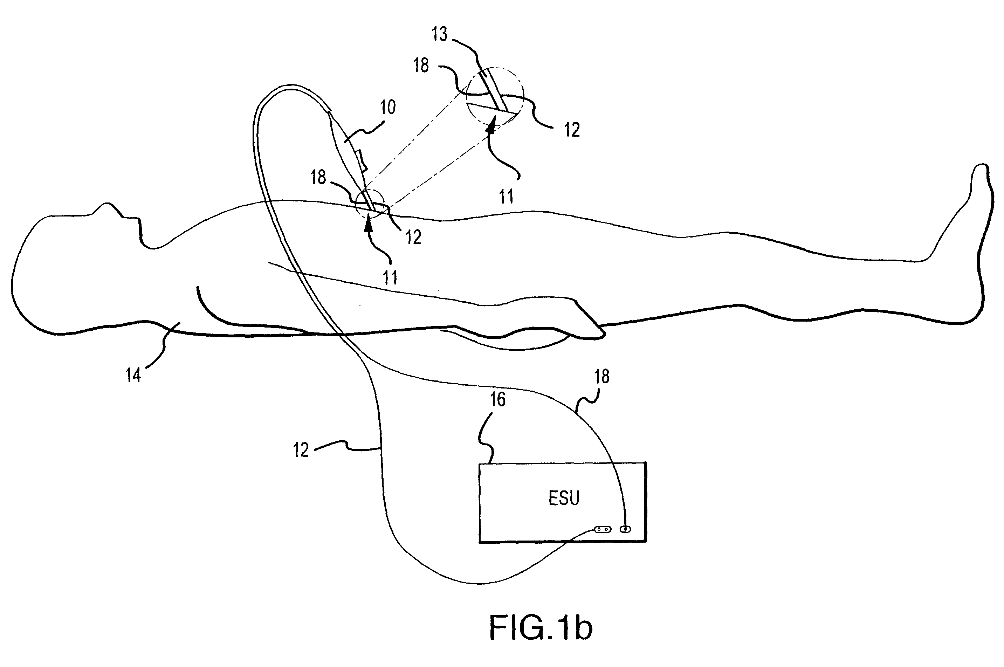

Electrosurgery performed on a patient using the multifunctional telescopic monopolar/bipolar surgical device of the present invention is shown in FIG. 1 b. With the present invention, the electrosurgery pencil 10 comprises a bipolar electrode 11 which comprises an active electrode 12 and a return electrode 18 which are separated by a material dielectric 13 which functions as a insulator. Both the active electrode 12 and the return electrode 18 are connected to the electrosurgery unit 1 6 thereby performing a completed circuit. When the electrosurgery unit 16 is activated and the bipolar electrode 11 touches the tissue of the patient 14, the circuit is closed through a very small portion of the patient's tissue between the active electrode 1 2 and the return electrode 18. This shortened distance between the active electrode 1 2 and return electrode 18 results in decrease of the power requirement from that previously needed in association with the monopolar electrode of the prior art in order to effectuate cutting and coagulation. This decreased distance between the two electrodes 12, 18 also results in decreasing the dangers associated with passing high voltages at high frequencies throughout a substantial portion of the patient's body, one of those risks being an increased possibility of burns to the patient.

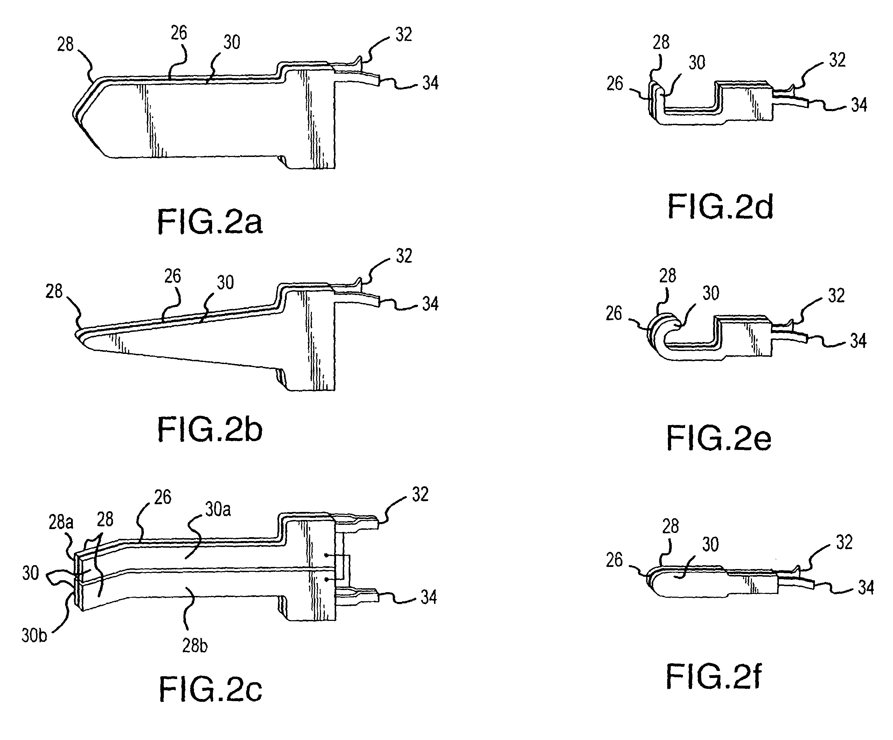

FIGS. 2a - c show perspective views of different embodiments of bipolar electrodes which comprise part of the multifunctional telescopic monopolar/bipolar surgical device of the present invention for open electrosurgery procedures. These electrodes are capable of exhibiting both bipolar and monopolar functioning in conjunction with an electrosurgery unit.

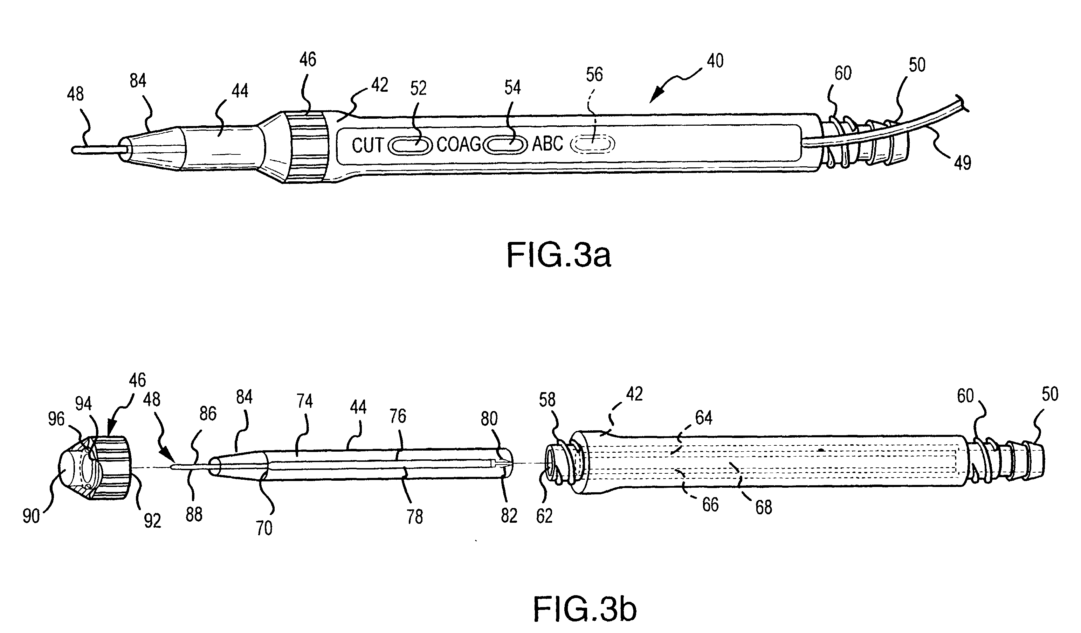

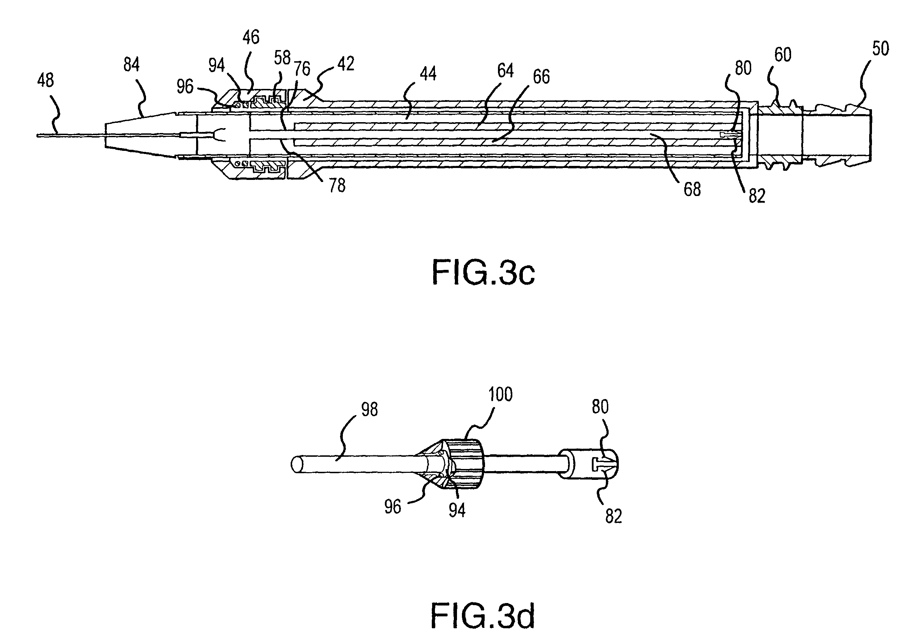

FIG. 2a represents a perspective view of a bipolar blade electrode. An insulator 26 is sandwiched between the active electrode 28 and the return electrode 30. Active electrode 28 further comprises an active prong 32 which is designed to come in contact with an active conductor contained within the telescopic element of the device of the present invention which is shown and described later with reference to FIGS. 3b and 3c. Further, the return electrode 30 comprises a return prong 34 which designed to make contact with a return conductor contained within the telescopic element of the device of the present invention which is also shown and described later with reference to FIGS. 3b - 3c.

FIG. 2b represents a perspective view of a bipolar needle electrode. Again, an insulator 26 is sandwiched between the active electrode 28 and the return electrode 30 wherein the active electrode 28 and return electrode 30 further comprise an active prong 32 and return prong 34, respectively, for connection to conductor elements contained within a telescopic element of a device of the present invention as shown in FIGS. b - 3c.

FIG. 2c represents a perspective view of a special single bipolar electrode that can be used for cutting and/or coagulation, as well as for surface coagulation or ablation. An insulator 26 is sandwiched between active and return electrodes wherein one of each of an active electrode 28 and a return electrode 30 are positioned on opposite sides of the insulator 26. More specifically, on a first side 36 of insulator 26 there is a top active electrode 28A and a bottom return electrode 30B and on a second side 38 of insulator 26 there is a top return electrode 3DA and a bottom active electrode 28B. Active prong 32 connects top active electrode 28A to bottom active electrode 288 while return prong 34 serves to connect top return electrode 30A to bottom return electrode 30B thereby forming connection means for the active electrode 28 and return electrode 30, respectively, when connecting the active and return electrodes 28, 30 to conductors contained within the telescopic elements of the device of the present invention as further explained with reference to FIGS. 3b - 3c. The previously described electrodes may further vary by comprising a variety of different shapes without detracting from the purpose of the invention.

FIGS. 2d - 2f depict perspective views of bipolar electrodes which comprise part of the multifunctional telescopic monopolar/bipolar surgical device of the present invention for performing endoscopic and/or laparoscopic procedures wherein the electrode is also capable of monopolar functioning alone. FIGS. 2d and 2e represent hook-shaped bipolar electrodes for use in endoscopic and laparoscopic procedures wherein the bipolar electrodes are also capable of functioning as monopolar electrodes. FIG. 2f shows a paddle-shaped bipolar electrode for use in endoscopic and/or laparoscopic procedures which may also function as a monopolar electrode. All of the bipolar electrodes depicted in FIGS. 2d - 2f comprise an insulator 26 which is sandwiched between an active electrode 28 and a return electrode 30. Further, the active electrode 28 comprises an active prong 32 while the return electrode 30 comprises a return prong 34 wherein both the active prong 32 and return prong 34 serve as connection means for connecting the active electrode 28 and return electrode 30 to conductors contained within a laparoscopic/endoscopic telescope element of the device of the present invention as further detailed in reference to FIG. 3d.

Other electrode shapes and forms may be used with respect to the bipolar electrodes for endoscopic and/or laparoscopic procedures without detracting from the purpose of the invention. Further, the active electrode 28 and return electrode 30 on all of the previously described bipolar electrodes in reference to FIGS. 2a - 2f may be reversed such that the active electrode 2B and return electrode 30 are on opposite sides of the insulator 26 than those on which they are depicted in the Figures.

Active electrode 28 is preferably comprised of stainless steel or other suitable conductors and return electrode 30 is preferably comprised of a ceramic or other suitable material which can function as an insulator. The insulator 26 is preferably comprised of a dielectric material which is suitable for use as a dielectric in radio frequency applications and at very high temperatures such as certain types of ceramics. However, any dielectric materials that can meet the conditions for RF applications at very high temperatures may be used.

As previously explained with reference to FIG. 1B, the circuit created by the active electrode 28 and return electrode 30 is closed when the ESU is activated and the electrode touches the tissue of a patient. This very small portion of the patient's tissue closes the gap between the active electrode 28 and the return electrode 30. As a result, much less power is needed to traverse the patient's tissue in order to close the circuit and effectuate the cutting and coagulation operations. A load resistor can be installed in series to avoid an accidental short circuit in between the two electrodes.

Turning now to FIG. 3a, there is shown a perspective view of the multifunctional telescopic monopolar/bipolar electrosurgical device of the present invention. In brief, the multifunctional telescopic monopolar/bipolar electrosurgical device 40 of the present invention comprises a main body 42, a telescopic body 44 circumferential contained within the main body 42 such that it can be extended outward from, and retracted into, the main body 42, a locking element 46 which locks the telescopic body 44 to the main body 42 at a predetermined extracted length, a bipolar electrode 48 contained within the telescopic body and main body such that it is capable of being in electrical contact with an electrosurgery unit, and a connector element 50 for connecting the main body 42 of the device 40 to a smoke evacuator tubing (not shown). The main body 42 of the device 40 further comprises a series of selection buttons, one selection button for cutting 52, one selection button for coagulation 54, and one selection button for argon beam coagulation 56. The selection button for argon beam coagulation is optional. The main body may be provided with only cutting and coagulation selection buttons 52, 54.

An exploded view of the multifunctional telescopic monopolar/bipolar electrosurgical device 40 of the present invention is illustrated in FIG. 3b. The main body 42 of the device 40 comprises a distal thread 55 and a proximal thread 60 which are connected by a channel 62. The main body 42 further comprises an active contact 64 and a return contact 66 which are located parallel to one another on an interior surface of the channel 62 having a gap located there between which resembles an elongated slot 68. The telescopic body 44 comprises a distal end 70 and a proximal end 72 which are separated by a second channel 74 that is smaller in diameter than the channel 62 contained within the main body 42. The telescopic body 44 further comprises a pair of elongated conductors 76, 78 located within the second channel 74 of the telescopic body 44. Each of the elongated conductors 76, 78 terminate in contact prongs 80, 82, respectively, such that the contact prongs 80, 82 are located on the external surface of the telescopic body 44 near its proximal end 72. A hollow nozzle 84 may be connected to the distal end 70 of the telescopic body 44.

The bipolar electrode 48 is connected to the telescopic body 44 such that the active electrode 86 and return electrode 88 are in contact with the elongated conductors 76, 78, respectively. Finally, the locking element 46 comprises a hollow interior having a distal end 90 and proximal end 92. The locking element 46 further comprises a ridge 94 contained within its hollow interior and an "O" ring 96 which is seated on the ridge 94. As a result, the locking element 46 can be slid over the telescopic body 44 and connected to the distal thread 58 of the main body 42 to enable the telescopic body 44 to be locked in position within the main body 42.

FIG. 3c shows a cross sectional view of the multifunctional telescopic monopolar/bipolar electrosurgery unit pencil shown in FIG. 3a. As previously described, the main body 42 contains active contact 64 and patient return contact 66 which are parallel to one another so that they are separated by the same distance along their entire lengths. Contact prongs 80, 82 contained on the external surface of the telescopic body 44 are slideably engaged and maintained within the elongated slot 68 which separates the active contact 64 from the patient return contact 66. The bipolar electrode 48 is connected to the contact prongs 80, 82 via the elongated conductors 76, 78. The telescopic body 44 is extracted or retracted within the main body 42 to adjust for the desired length of the bipolar electrode 48.

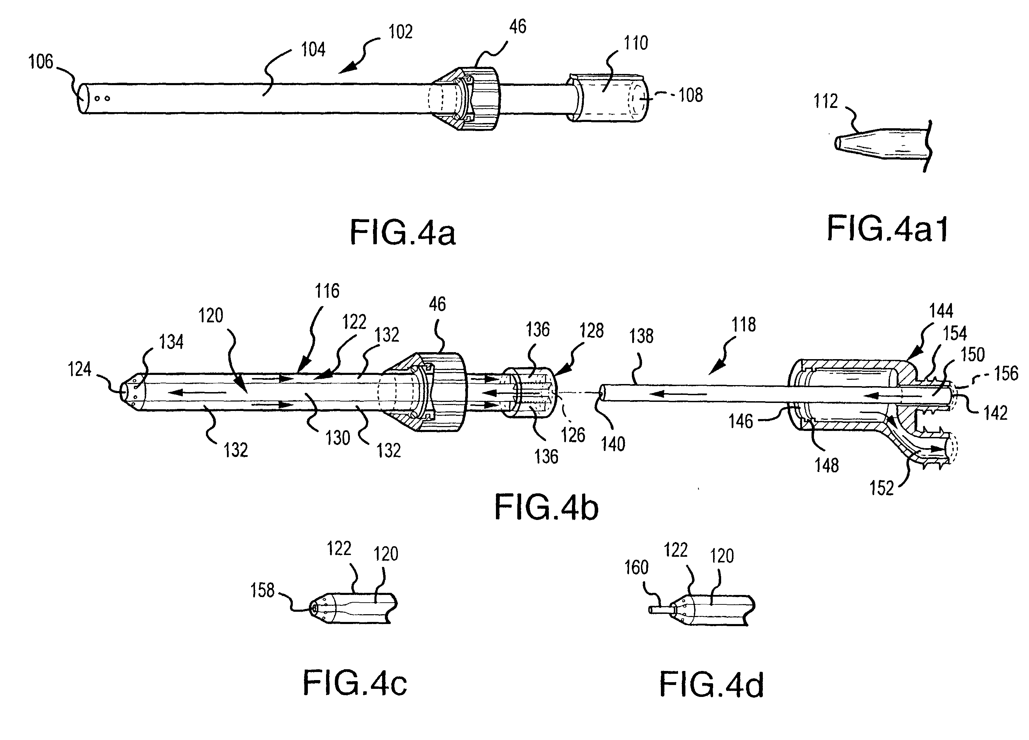

Argon beam coagulation attachments (not shown) replace the telescopic body 44 described above with a special telescope and end attachment similar to those described in my previous patent application entitled "A Telescopic Surgical Device and Method Therefor" may be attached to the proximal thread 60 of the main body 42 of the device 40. A suction/irrigation attachment such as that later described and shown in FIGS. 4a - 4b, as well as a connector for smoke evacuation tubing (not shown) may also be connected to proximal thread 60 of the main body 42 of the device 40. The locking element 46 is threaded onto the distal thread 58 of the main body 42 of the device 40 in order to lock the telescopic body 44 in place. The telescopic body 44 is locked in position by tightening the locking element 46 against the distal thread 58 of the main body 42. As a result, the ridge 94 contained within the locking element 46 is pushed forward such that it presses over the "O" ring 96 which in turn presses over the telescopic body 44 thereby locking it in place.

During use, when either the cutting button 52 or the coagulation button 54 is depressed, the active contact 64 is energized and the return contact 66 acts to return current from the patient to the electrosurgery unit. Contact prongs 80, 82 function to pass the radio frequency energy through elongated conductor 76 to the bipolar electrode 48 and then return the RF energy through elongated conductor 78 back to contact prong 82. If the multifunctional telescopic monopolar/bipolar surgical pencil device of the present invention is used for monopolar only, then the active contact 64 and return contact 66 will both conduct the active energy and a separate return electrode will be provided and utilized as represented in FIG. 1A. Energy is brought to the multifunctional telescopic monopolar/bipolar surgical pencil device of the present invention by electrical cord 49 (See FIG. 3a) which is connected to an electrosurgery unit (not shown).

Turning now to FIG. 3d, there is shown an endoscopic/laparoscopic telescopic body 98 which replaces the telescopic body 44 shown in FIGS. 3b - 3c in order to perform endoscopic and/or laparoscopic procedures. The second locking element 100 works similar to the locking element 46 described with reference to FIGS, 3b - 3c. The cutting operation is activated when the cutting button 52 is depressed and coagulation is activated when the coagulation button 54 is depressed. Argon beam coagulation button number 56 is optional and, if used, the endoscopic/laparoscopic telescope body 98 is replaced with a different telescope such as that described in my pending patent application entitled "A Telescopic Surgical Device and Method Therefor" and an adaptor is attached to the end of the device 40 at the proximal thread 60 of the main body 42 of the device 40. The adaptor is very similar to the adaptor for suction/irrigation that is later shown and described with reference to FIG. 4.

FIGS. 4a - 4c show another embodiment of the electrosurgery pencil of the present invention which is directed toward a telescopic suction/irrigation pencil for electrosurgery. The telescopic monopolar/bipolar surgical pencil depicted and described in FtGS. 3a - 3d will become a telescopic suction/irrigation pencil by replacing the telescopic body 14 in FIGS, 3b - 3c and the endoscopiellaparoscopic telescopic body 98 in FIG. 3d with the attachments presented in FIGS. 4a - 4b. The resulting telescopic suction/irrigation pencil is advantageous in that it permits the surgeon to adjust the lengths of the suction/irrigation tip without the need for removing and reattaching tips of different lengths.

FIG. 4a shows a perspective view of a first embodiment of a suction/irrigation attachment 102 comprising a singular hollow tube 104 having a distal end 106 and a proximal end 108. The suction/irrigation attachment 102 further comprises a sliding guide 110 which is circumferentially fit about the proximal end 108 of the singular hollow tube 104 thereby allowing the suction/irrigation attachment 102 to be slidably engaged within the main body 42 of the telescopic monopolar/bipolar electrosurgery device 40 shown in FIG. 3b. The locking element 46 shown in FIG. 3b is also used in conjunction with the suction/irrigation attachment 102 shown in FIG. 4a in order to lock the suction/irrigation attachment 102 in place at a predetermined extracted length from the main body 42 shown in FIG. 3b. The resulting device is a telescopic suction/irrigation pencil for use with electrosurgery as well as laparoscopic and endoscopic procedures.

Suction and irrigation are activated by using the cutting button 52 shown in FIG. 3a for irrigation and the coagulation button 54 shown in FIG. 3a for suction. During irrigation, the irrigation fluid enters the telescopic suction/irrigation pencil through connector element 50, then passes through the proximal end 108 of the singular hollow tube 104 shown in FIG. 4a and then exits the telescopic suction/irrigation pencil through the distal end 106 of the singular hollow tube 104 shown in FIG. 4a. in contrast, when suction is activated, fluid is drawn from the patient, and enters the telescopic suction/irrigation pencil at the distal end 106 of the singular hollow tube 104, is further drawn past the proximal end 108 of the singular hollow tube 104, and finally exits the telescopic suction/irrigation pencil through the connector element 50 shown in FIG. 3b.

The distal end 106 of the suction/irrigation attachment 102 may be narrowed to form a nozzle 112 such as that shown in FIG. 4a. The nozzle 112 configuration of the distal end 106 facilitates the use of the telescopic suction/irrigation pencil for hydrodissection by enabling the irrigation fluid to exit the distal end 106 of the suction/irrigation attachment 102 with high pressure and velocity,

FIG. 4b illustrates an exploded perspective view of a second embodiment of a suction/irrigation attachment 114 which is capable of performing suction and irrigation simultaneously, The lengths of the telescopic suction and irrigation tubes may also be adjusted. The second embodiment of the suction/irrigation attachment 114 comprises a double channeled tube 116 and a singular connection tube 118. The double channeled tube 116 comprises a first hollow tube 120 circumferentially contained within a second hollow tube 122 wherein the first and second hollow tubes 120, 122 are of substantial the same length. The double channeled tube 116 further includes a distal end 124, a proximal end 126, a spacer element located at the proximal end 126 of the double channeled tube 116, and a locking element 46 which functions like the locking element 46 shown and described with reference to FIG. 3b.

The double channeled tube 116 contains a central inner channel 130 which is defined by the hollow inner area of the first hollow tube 120 and an outer ring-shaped channel 132 which is defined by the opening generated by the difference between the inner diameter of the second hollow tube 122 and the outer diameter of the inner hollow tube 120. irrigation is conducted through the central inner channel 130 while suction is conducted through the outer ring-shaped channel 132.

The second hollow tube 122 is closed about the first hollow tube 120 at the proximal end 126 of the double channeled tube 116 but a plurality of apertures 134 are contained about the circumference of the second hollow tube 122 near proximal end 126 thereby creating an opening through which fluid can pass from the outer ring-shaped channel 132. The spacer element 128 comprises a shortened hollow cylinder having a proximal open end and a plurality of wings 136 located within its opposite open end. The plurality of wings 136 are positioned within the outer ring-shaped channel 132 located between the first hollow tube 120 and the second hollow tube 122 to maintain the distance between the tubes 120, 122 and support the outer ring-shaped channel 132 while still allowing irrigation fluid to pass through the outer ring-shaped channel 132 with minimal resistance.

The singular connection tube 118 comprises a tube 138 having an open distal end 140 and an open proximal end 142 wherein the open proximal end 142 is seated within a multi-connector piece 144. Multi-connector piece 144 comprises a hollow piece having a large open distal end 146 with grooves 148 for receiving threads and two smaller channels 150, 152 each having threads 154 located at its proximal end 156. Threaded channel 150 is designed to be attached to an irrigation means while threaded channel 152 is designed for attachment to a suctioning means.

During use, the grooves 148 at the large open distal end 146 of the multi-connector piece 144 are connected to the proximal thread 60 of the main body 42 of the device 40 shown in FIG. 3b while the tube 138 is inserted through the channel 62 of the main body 42 such that tube 139 will exit the channel 62 and enter the central inner channel 130 of the first hollow tube 120 of the double channeled tube 116 when the spacer element 128 is slid within the main body 42 of the device 40 shown in FIG. 3b. Tube 138 has an outer diameter that is slightly smaller than the inner diameter of the first hollow tube 120 so that hollow tube 120 and tube 138 form a telescope when concentrically enjoined.

When irrigation is activated, irrigation fluid will be introduced under pressure through the open proximal end 142 of tube 138 and will traverse the telescopic configuration resulting from joining tube 138 inside of first hollow tube 120, and will then exit at the distal end 124 of the double channeled tube 116 through the first hollow tube 120. When suction is activated, suctioning means is applied at suctioning channel 152 and fluid is drawn from the patient through the plurality of apertures 134 contained within the second hollow tube 122. The fluid is drawn through the ring-shaped outer channel 132 and then exits at the suctioning channel 152. Spacer element 128 slides within the main body 42 of the device 40 shown in FIG. 3b and the second hollow tube 122 is locked into place by connecting locking element 45 to distal thread 58 of the main body 42 of the device 40.

The distal end 124 of the double channeled tube 116 of the suction/irrigation attachment shown in FIG. 4b may comprise an alternative configuration such as that shown in FIGS. 4c - 4d. FIGS. 4c-4d depict a nozzle configuration in which the distal open end 158 of first hallow tube 120 forms a needle nose 158 which is capable of being extracted and retracted from within the second hollow tube 122 thereby allowing the operator or surgeon to adjust the length of the irrigation means such that it extends beyond the length of the suctioning means of the cite of application.

While the invention has been particularly shown and described with reference to the preferred embodiments thereof, it will be understood by those skilled in the art that changes in form and detail of the foregoing may be made while still falling within the claims of the present invention.

## Claims

1. A telescopic electrosurgery pencil (40) for both laparoscopic and endoscopic procedures wherein said electrosurgery pencil (40) comprises;
a hollow tubular shaped body (44) having an electrode element (48) comprising an active electrode (28,86), a return electrode (30,88) and an insulator (26) sandwiched between the active electrode (28,86) and the return electrode (30,88),
a hollow tubular shaped handpiece (42) having two electrical contacts (64,66) contained therein wherein the electrode element (48) is connected to the electrical contacts (64,66) of the handpiece (42) and the electrical contacts (64,66) of the handpiece (42) are connectable to an energy source,
said contacts (64,66) being located parallel to one another separated by the same distance along an interior length of said handpiece (42) by an elongated slot (68); and
said body (44) being telescopic within said handpiece (42) and comprising first and second elongated conductors (76,78) in contact with said active and return contacts (64,66), respectively; wherein the active electrode (28,86) and the return electrode (30,88) are in contact with first and second elongated conductors (76,78) respectively, each of said first and second elongated conductors (76,78) terminating in contact prongs (80,82) contained on the external surface of the telescopic body (44), which prongs are slideably engaged and maintained within said elongated slot (68),
whereby the telescopic body (44) may be extracted or retracted within the handpiece (42).

2. The telescopic electrosurgery pencil (40) of claim 1, further comprising locking means (46) for locking the hollow tubular shaped telescopic body (44) in a given position in relation to the hollow tubular shaped handpiece (42).

3. The telescopic electrosurgery pencil (40) of claim 1, wherein said hollow tubular shaped handpiece (42) comprises means for alternately switching between cutting and coagulation (52,54) using the electrode tip (48).

4. The telescopic electrosurgery pencil (40) of claim 1, further comprising smoke evacuation means (104,132) for removing smoke and debris produced near the electrode tip (48) during a medical procedure using said electrosurgery pencil (40) and an energy,source.

5. The telescopic electrosurgery pencil (40) of claim 1, wherein said handpiece (42) includes an open distal end (58) and an open proximal end (60) connected by a channel (62), at least one of the electrical contacts (64,66) located along an interior of the handpiece (42), and connection means (49) for connecting the handpiece (42) to the energy source for alternately activating suction and irrigation functions.

6. The telescopic electrosurgery pencil (40) of claim 5, wherein said telescopic body (44) includes distal and proximal ends (70,72) wherein the distal open end (70) of the telescopic body (44) is introduced into the open proximal end (60) of the handpiece (42) such that the telescopic body (44) is concentrically retained within the channel (62) of the handpiece (42).

7. The telescopic electrosurgery pencil (40) of claim 6, further comprising a telescopic suction/irrigation apparatus (114) wherein said telescopic body (44) is a double channeled tube (116) comprising an inner channel (130) and an outer channel (132).

8. The telescopic electrosurgery pencil (40) of claim 7, wherein said suction/irrigation apparatus (114) includes a singular elongated tube (118) having an open distal end (140) and an open proximal end (142), and a multi-connector piece (144), wherein the open proximal end to said singular elongated tube (118) is seated within the multi-connector piece (144), and the open distal end (140) of the singular elongated tube (118) is introduced into the proximal open end (126) of the inner channel (120) to said double channeled telescopic body (116) such that the singular elongated tube (118) is concentrically retained within the inner channel (120) of the double channeled telescopic body (116), and wherein the open proximal end (60) of the handpiece (42) is connected to the multi-connector piece (144) which has a separate entrance port (150) for irrigation and a separate exit port (152) to suctioning means.

9. The telescopic electrosurgery pencil (40) of claim 8, further comprising nozzle means (112) wherein an inner tubular member which defines the inner channel (120) is extractable and retractable along a length of an outer tubular member which defines the outer channel (122).

10. The telescopic electrosurgery pencil (40) of claim 1, further comprising means for effectuating at least one of alternate and simultaneous suction and irrigation functions.

11. The telescopic electrosurgery pencil (40) of claim 10, further comprising means for automatically retracting said electrode tip (48) upon activation of said means for effectuating at least one of alternate and simultaneous suction and irrigation functions.

12. The telescopic electrosurgery pencil (40) of any one of the preceding claims, wherein said active electrode (28,86) functions as a monopolar electrode.

13. The telescopic electrosurgery pencil (40) of any one of the preceding claims, wherein said handpiece (42) comprises means for alternately switching between cutting (52) and coagulation (54) using the electrode tip (48).

14. The telescopic electrosurgery pencil (40) of any one of the preceding claims, further comprising means for performing at least one of cutting, coagulation, and argon beam coagulation (52,54,56).

## Patentansprüche

1. Teleskopischer elektrochirurgischer Stift (40) für sowohl laparoskopische als auch endoskopische Verfahren, wobei der elektrochirurgische Stift (40) folgendes umfaßt: einen hohlen röhrenförmigen Körper (44) mit einem Elektrodenelement (48), das eine aktive Elektrode (28, 86), eine Rückelektrode (30, 88) und einen Isolator (26) umfaßt, der zwischen der aktiven Elektrode (28, 26) und der Rückelektrode (30, 88) eingeschlossen ist,
ein hohles röhrenförmiges Handstück (42) mit zwei elektrischen Kontakten (64, 66), die darin enthalten sind, wobei das Elektrodenelement (48) mit den zwei elektrischen Kontakten (64, 66) des Handstücks (42) verbunden ist und die elektrischen Kontakte (64, 66) des Handstücks (42) mit einer Energiequelle verbunden werden können,
wobei die Kontakte (64, 66) parallel zueinander um den gleichen Abstand entlang einer Innenlänge des Handstücks (42) durch einen länglichen Schlitz (68) beabstandet angeordnet sind, und
der Körper (44) in dem Handstück (42) teleskopisch ist und einen ersten und einen zweiten länglichen Leiter (76, 78) aufweist, die in Kontakt mit dem Aktiv- bzw. Rückkontakt (64, 66) sind, wobei die aktive Elektrode (28, 86) und die Rückelektrode (30, 88) in Kontakt mit dem ersten bzw. dem zweiten länglichen Leiter (76, 78) sind, und der erste und zweite längliche Leiter (76, 78) in Kontaktzungen (80, 82) enden, die an der äußeren Oberfläche des teleskopischen Körpers (44) enthalten sind, wobei die Zungen mit dem länglichen Schlitz (68) gleitfähig zusammenwirken und darin gehalten werden,
wobei der teleskopische Körper (44) in dem Handstück (42) ausgefahren oder eingefahren werden kann.

2. Teleskopischer elektrochirurgischer Stift (40) nach Anspruch 1, der weiterhin eine Verriegelungseinrichtung (46) zum Verriegeln des hohlen röhrenförmigen teleskopischen Körpers (44) in einer vorgegebenen Position gegenüber dem hohlen röhrenförmigen Handstück (42) umfaßt.

3. Teleskopischer elektrochirurgischer Stift (40) nach Anspruch 1, bei dem das hohle röhrenförmige Handstück (40) eine Einrichtung zum Umschalten zwischen Schneiden und Koagulieren (52, 54) unter Verwendung der Elektrodenspitze (48) aufweist.

4. Teleskopischer elektrochirurgischer Stift (40) nach Anspruch 1, der weiterhin eine Rauchabsaugeinrichtung (104, 132) zum Entfernen von Rauch und Trümmern umfaßt, die nahe der Elektrodenspitze (48) bei einem medizinischen Verfahren unter Verwendung des elektrochirurgischen Stifts (40) und einer Energiequelle erzeugt werden.

5. Teleskopischer elektrochirurgischer Stift (40) nach Anspruch 1, bei dem das Handstück (42) ein offenes distales Ende (48) und ein offenes proximales Ende (60), die über einen Kanal (62) verbunden sind, wobei wenigstens einer der elektrischen Kontakte (64, 66) entlang eines Inneren des Handstücks (42) angeordnet ist, und eine Verbindungseinrichtung (49) zum Verbinden des Handstücks (42) mit der Energiequelle zum alternativen Aktivieren einer Saug- und einer Spül-Funktion aufweist.

6. Teleskopischer elektrochirurgischer Stift (40) nach Anspruch 5, bei dem der teleskopische Körper (44) ein distales und ein proximales Ende (70, 72) aufweist,
wobei das distale offene Ende (70) des teleskopischen Körpers (44) in das offene proximale Ende (60) des Handstücks (42) so eingeführt ist, daß der teleskopische Körper (44) konzentrisch in den Kanal (62) des Handstücks (42) zurückgehalten wird.

7. Teleskopischer elektrochirurgischer Stift (40) nach Anspruch 6, der weiterhin eine teleskopische Absaug-/Spül-Vorrichtung (114) umfaßt, wobei der teleskopische Körper (44) eine Zweikanalröhre (116) ist, die einen inneren Kanal (130) und einen äußeren Kanal (132) umfaßt.

8. Teleskopischer elektrochirurgischer Stift (44) nach Anspruch 7, bei dem die Absaug-/Spül-Vorrichtung (114) eine einzelne längliche Röhre (118) mit einem offenen distalen Ende (140) und mit einem offenen proximalen Ende (142) und ein Mehrfachverbindungsstück (144) umfaßt, wobei an das offene proximale Ende der einzelnen länglichen Röhre (118) in dem Mehrfachverbindungsstück (144) eingeführt ist, und das offenen distale Ende (140) der einzelnen länglichen Röhre (118) in das proximale offene Ende (126) des inneren Kanals (120) des teleskopischen Zweikanalkörpers (116) so eingeführt ist, daß die einzelne längliche Röhre (118) konzentrisch in dem inneren Kanal (120) des teleskopischen Zweikanalkörpers (116) zurückgehalten wird, und bei dem offene proximale Ende (60) des Handstücks (42) mit dem Mehrfachverbindungsstück (144) verbunden ist, das einen separaten Eingangseinschluß (150) zum Spülen und einem separaten Ausgangsanschluß (152) für eine Absaugeinrichtung aufweist.

9. Teleskopischer elektrochirurgischer Stift (40) nach Anspruch 8, der weiterhin eine Düseneinrichtung (112) aufweist, wobei ein inneres röhrenförmiges Element, welches den inneren Kanal (120) festlegt, entlang einer Länge eines äußeren röhrenförmigen Elements, welches den äußeren Kanal (122) festlegt, ausfahrbar und zurückfahrbar ist.

10. Teleskopisch elektrochirurgischer Stift (40) nach Anspruch 1, der weiterhin eine Einrichtung zum Bewirken von wenigstens einer wechselnden und gleichzeitigen Saug- und Spülfunktion umfaßt.

11. Teleskopisch elektrochirurgischer Stift (40) nach Anspruch 10, der weiterhin eine Einrichtung zum automatischen Zurückfahren der Elektrodenspitze (84) aufgrund der Aktivierung der Einrichtung zur Bewirkung der wenigstens einen wechselnden und gleichzeitigen Saug- und Spülfunktion umfaßt.

12. Teleskopischer elektrochirurgischer Stift (40) nach einem der vorhergehenden Ansprüche, bei dem die aktive Elektrode (28, 26) als eine monopolare Elektrode wirkt.

13. Teleskopischer elektrochirurgischer Stift (40) nach einem der vorhergehenden Ansprüche, bei dem das Handstück (42) eine Einrichtung zum Umschalten zwischen Schneiden (52) und Koagulieren (54) unter Verwendung der Elektrodenspitze (48) umfaßt.

14. Teleskopischer elektrochirurgischer Stift (40) nach einem der vorhergehenden Ansprüche, der weiterhin eine Einrichtung zum Ausführen von wenigstens eines der folgenden umfaßt: Schneiden, Koagulieren und ArgonstrahI-Koagulieren (52, 54, 56).

## Revendications

1. Stylet électrochirurgical télescopique (40) destiné à des processus laparoscopique et endoscopique, ledit stylet électrochirurgical (40) comportant :
un corps creux de forme tubulaire (44) ayant un élément d'électrode (48) comportant une électrode active (26, 86), une électrode de référence (30, 88) et un isolant (26) enserré entre l'électrode active (26, 86) et l'électrode de référence (30, 88),
une pièce à main creuse de forme tubulaire (42) ayant deux contacts électriques (64, 66) contenus dans celle-ci, l'élément d'électrode (48) étant connecté aux contacts électriques (64, 66) de la pièce à main (42), et les contacts électriques (64, 66) de la pièce à main (42) pouvant être connectés à une source d'énergie,
lesdits contacts (64, 66) étant positionnés parallèles l'un à l'autre séparés par la même distance le long d'une longueur intérieure de ladite pièce à main (42) par une fente allongée (68), et
ledit corps (44) étant télescopique dans ladite pièce à main (42), et comportant des premier et second conducteurs allongés (76, 78) en contact avec lesdits contacts actifs et de référence (64, 66), respectivement, l'électrode active (26, 86) et l'électrode de référence (30, 88) étant en contact avec les premier et second conducteurs allongés (76, 78), respectivement, chacun desdits premier et second conducteurs allongés (76, 78) se terminant en des griffes de contact (80, 82) contenues sur la surface extérieure du corps télescopique (44), lesquelles griffes étant mises en prise de manière coulissante et maintenues dans ladite fente allongée (68),
de sorte que le corps télescopique (44) peut être extrait de la pièce à main (42), ou rétracté dans celle-ci.

2. Stylet électrochirurgical télescopique (40) selon la revendication 1, comportant en outre des moyens de verrouillage (46) pour verrouiller le corps télescopique creux de forme tubulaire (44) dans une position donnée par rapport à la pièce à main creuse de forme tubulaire (42).

3. Stylet électrochirurgical télescopique (40) selon la revendication 1, dans lequel ladite pièce à main creuse de forme tubulaire (42) comporte des moyens pour commuter en alternance entre une découpe et une coagulation (52, 54) en utilisant la pointe d'électrode (48).

4. Stylet électrochirurgical télescopique (40) selon la revendication 1, comportant en outre des moyens d'évacuation de fumée (104, 132) pour enlever de la fumée et des débris produits à proximité de la pointe d'électrode (48) pendant un processus médical utilisant ledit stylet électrochirurgical (40) et une source d'énergie.

5. Stylet électrochirurgical télescopique (40) selon la revendication 1, dans lequel ladite pièce à main (42) comporte une extrémité distale ouverte (58) et une extrémité proximale ouverte (60) connectées par un canal (62), au moins un des contacts électriques (64, 66) positionné le long d'un intérieur de la pièce à main (42), et des moyens de connexion (49) pour connecter la pièce à main (42) à la source d'énergie pour activer en variante des fonctions d'aspiration et d'irrigation.

6. Stylet électrochirurgical télescopique (40) selon la revendication 5, dans lequel ledit corps télescopique (44) comporte des extrémités distale et proximale (70, 72), dans lequel l'extrémité distale ouverte (70) du corps télescopique (44) est introduite dans l'extrémité proximale ouverte (60) de la pièce à main (42), de telle sorte que le corps télescopique (44) est retenu de manière concentrique dans le canal (62) de la pièce à main (42).

7. Stylet électrochirurgical télescopique (40) selon la revendication 6, comportant en outre un dispositif d'aspiration/irrigation télescopique (114), dans lequel ledit corps télescopique (44) est un tube à deux canaux (116) comportant un canal intérieur (130) et un canal extérieur (132).

8. Stylet électrochirurgical télescopique (40) selon la revendication 7, dans lequel ledit dispositif d'aspiration/irrigation (114) comporte un tube allongé unique (118) ayant une extrémité distale ouverte (140) et une extrémité proximale ouverte (142), et une pièce à plusieurs connecteurs (144), dans lequel l'extrémité proximale ouverte dudit tube allongé unique (118) est mise en place dans la pièce (144) à plusieurs connecteurs, et l'extrémité distale ouverte (140) du tube allongé unique (118) est introduite dans l'extrémité proximale ouverte (126) du canal intérieur (120) vers ledit corps télescopique à deux canaux (116), de telle sorte que le tube allongé unique (118) est retenu de manière concentrique dans le canal intérieur (120) du corps télescopique à deux canaux (116), et dans lequel l'extrémité proximale ouverte (60) de la pièce à main (42) est connectée à la pièce (144) à plusieurs connecteurs qui a un orifice d'entrée séparé (150) pour irrigation, et un orifice de sortie séparé (152) vers des moyens d'aspiration.

9. Stylet électrochirurgical télescopique (40) selon la revendication 8, comportant en outre des moyens formant buse (112), dans lequel un élément tubulaire intérieur qui définit le canal intérieur (120) peut être extrait et peut être rétracté le long d'une longueur d'un élément tubulaire extérieur qui définit le canal extérieur (122).

10. Stylet électrochirurgical télescopique (40) selon la revendication 1, comportant en outre des moyens pour effectuer au moins une parmi des fonctions d'aspiration et d'irrigation alternées et simultanées.

11. Stylet électrochirurgical télescopique (40) selon la revendication 10, comportant en outre des moyens pour rétracter automatiquement ladite pointe d'électrode (48) lors de l'actionnement desdits moyens pour effectuer au moins une des fonctions d'aspiration et d'irrigation alternées et simultanées.

12. Stylet électrochirurgical télescopique (40) selon l'une quelconque des revendications précédentes, dans lequel ladite électrode active (28, 86) fonctionne comme une électrode monopolaire.

13. Stylet électrochirurgical télescopique (40) selon l'une quelconque des revendications précédentes, dans lequel ladite pièce à main (42) comporte des moyens pour une commutation en alternance entre une découpe (52) et une coagulation (54) en utilisant la pointe d'électrode (48).

14. Stylet électrochirurgical télescopique (40) selon l'une quelconque des revendications précédentes, comportant en outre des moyens pour effectuer au moins une opération parmi une découpe, une coagulation et une coagulation sous faisceau d'argon (52, 54, 56).
